# EUROPÄISCHE PATENTANMELDUNG

(11) **EP 0 700 694 A1**
(43) Veröffentlichungstag der Anmeldung: **13.03.1996**
(21) Anmeldenummer: 94113968.5
(22) Anmeldetag: 06.09.1994
(51) Int. Cl.: A63B 24/00

(54) **Trainings- und Diagnoseverfahren**

(71) Anmelder: Rudolf Presl GmbH & Co. Klinik Bavaria Rehabilitations KG, D-01731 Kreischa (DE)
(72) Erfinder: Blümel, Georg, Doz. Dr. sc. nat.,, 04328 Leipzig (DE); Köllner, Jürgen, Dr. paed.,, 04279 Leipzig (DE)
(74) Vertreter: von Samson-Himmelstjerna, Friedrich R., Dipl.-Phys.

(57) **Zusammenfassung**

Die vorliegende Erfindung beschreibt ein Verfahren zur Diagnose der Fähigkeiten des menschlichen Bewegungsapparates und des kontrollierten und dosierten Trainings dieser Fähigkeiten unter Verwendung eines um mindestens eine Steckkarte (16) erweiterten herkömmlichen Rechners (14), eines herkömmlichen Trainingsgeräts (60) und eines Meßwertaufnehmers (18). Dabei werden zunächst Startparameter am Trainingsgerät (60) und am Rechner (14) eingegeben. Ein Meßwertaufnehmer (18) wird mit dem Traingsgerät (60) und dem Rechner (14) verbunden. Die aufgrund einer von einem Probanden (20) ausgeführten Bewegung vom Meßwertaufnehmer (18) erzeugten Daten werden an die zusätzliche Steckkarte (16) des Rechners (14) übertragen. Simultan mit der momentanen Bewegung des Probanden (20) wird diesem eine daraus abgeleitete Größe vermittelt (10,32). Aus den Daten werden im Rechner (14) verschiedene zur Beurteilung des Trainings bzw. der Diagnose geeignete Parameter berechnet. Die Ergebnisse der Auswertung werden anschließend an einem Bildschirm (10) angezeigt.

## Beschreibung

Die vorliegende Erfindung betrifft ein Verfahren zur Diagnose der Fähigkeiten des menschlichen Bewegungsapparates und des kontrollierten und dosierten Trainings dieser Fähigkeiten.

Bekannt sind Trainings- und Diagnoseverfahren, bei denen zur Aufzeichnung von Bewegungen bzw. bewegungsproportionalen Größen, die ein Proband an einem dazu geeigneten Gerät ausführt, Datenverarbeitungsanlagen (DV) verwendet werden.

Allerdings werden dazu meist keine herkömmlichen, somit preiswerten DV, beispielsweise Personal Computer (PC), und herkömmliche, in jedem Fitness-Center anzutreffende, preiswerte Trainingsgeräte verwendet, sondern speziell aufeinander abgestimmte, damit teuere Kombinationen aus DV und Trainingsgerät. Vielfach ist bereits in einem Trainingsgerät eine DV implementiert, wobei aufgrund der geringen Stückzahlen diese Geräte teuer sind und deren Einsatz nur kapitalstarken Unternehmen möglich ist, nicht jedoch kleineren Einrichtungen oder privaten Anwendern.

Ein weiterer Nachteil der aus dem Stand der Technik bekannten Trainings- und Diagnoseverfahren liegt darin, daß die zu einer von einem Probanden ausgeführten Bewegung proportionale Anzeige erst nach der Ausführung einer Übung an einem Rechner-Bildschirm einzusehen ist. Somit kann der Proband nicht schon während der Durchführung einer Übung seine Krafteinteilung dosieren bzw. koordinieren. Er handelt vielmehr ohne Kontrolle, frei nach Gefühl.

Darüber hinaus ist die Anzeige dann gewöhnlich nur vom Therapeuten einsehbar, nicht aber vom Probanden.
Daraus ergibt sich ein weiterer Nachteil: Beispielsweise weiß der Proband bei den herkömmlichen Trainingsverfahren, die ja auch bei der Rehabilitation verletzter Extremitäten Anwendung finden, nicht, in welchem Bereich er schmerzfrei üben kann. Er kann sich also, aus Angst vor einem plötzlichen Schmerz, nicht kontrolliert an seine Schmerzgrenze herantasten und diese dann auf dem Wege der Rehabilitation dosiert ausweiten.

Aufgabe vorliegender Erfindung ist es daher, ein Verfahren zur Diagnose und zum Training der Fähigkeiten des menschlichen Bewegungsapparates bereitzustellen, bei dem eine gleichzeitige Kontrolle des Bewegungsablaufs und des Kraftaufwands durch den Probanden gewährleistet ist.

Zur Lösung dieser Aufgabe dienen die Merkmale des unabhängigen Anspruches.

Vorteilhafte Ausgestaltungen sind in den Unteransprüchen beschrieben.

Durch das erfindungsgemäße Verfahren wird eine sogenannte Feedback-Technik bereitgestellt, wobei in vorteilhafter Weise beliebige herkömmliche Trainingsgeräte in kürzester Zeit ohne großen Aufwand für diese Technik aufgerüstet werden können.

In Verbindung mit der Bestimmung der vom Probanden umgesetzten Leistung ist somit jedes herkömmliche Trainingsgerät zu einem Ergometer umwandelbar.

Mittels des erfindungsgemäßen Diagnoseverfahrens kann der Bewegungsbereich, d.h. die während einer Beuge-Streck-Bewegung gegenüber einem Grundzustand ausgeführte Weg- bzw. Winkeländerung, einer beispielsweise verletzten Extremität ermittelt werden, innerhalb dessen der Proband diese Extremität schmerzfrei bewegen kann. Durch Anzeigen einer der maximalen Änderung bzw. Bewegungsamplitude proportionalen Größe an einem Bildschirm kann sich der Proband dieser Grenze kontrolliert und schmerzfrei annähern.

Durch Ermittlung des maximalen Bewegungsbereichs der gesunden Extremität unter Anwendung des erfindungsgemäßen Verfahrens kann die kranke mit der gesunden Extremität verglichen werden; eine Vorgabe des Bewegungsbereichs der gesunden Extremität als Zielgröße für den Bewegungsbereich der kranken Extremität ist möglich.

Weiterhin ist äußerst vorteilhaft, daß der Erfolg des Trainings in einfacher Weise quantitativ bestimmbar ist.

In einer weiteren vorteilhaften Ausführungsform muß der Proband seine Bewegung so koordinieren, daß die dadurch bewirkte Anzeige, die seiner Bewegung proportional ist, am Bildschirm möglichst exakt mit einer vom Therapeuten ausgewählten Vorgabekurve zusammenfällt. Dadurch kann der Therapeut dem Probanden in einfacher Weise vermitteln, in welcher Form, z.B. mit welcher Amplitude innerhalb welcher Zeit, der Proband seine Beuge-Streck-Bewegungen durchzuführen hat. Durch Wahl geeigneter Formen dieser Vorgabekurve lernt der Proband seine Kraft kontrolliert und koordiniert einzusetzen.

Durch Einsatz mehrerer Meßwertaufnehmer ist die räumliche Erfassung und Analyse eines Bewegungsablaufs möglich. Dies ist besonders vorteilhaft beim Training technischer Sportdisziplinen, z.B. beim Speerwurf.

Beim erfindungsgemäßen Verfahren kann der Proband alternativ gegen die Schwerkraft, gegen eine Federkraft oder gegen eine motorisch erzeugte Kraft arbeiten.

Die Vermittlung einer seiner aktuellen Bewegung proportionalen Größe ist bereits während der Durchführung einer Übung über verschiedene Sinnesorgane des Probanden möglich. So kann sie auf visuellem Wege dem Auge, auf akustischem Wege dem Ohr oder über den Druck der Haut vermittelt werden.

In einer weiteren vorteilhaften Ausführungsform wird die vom Probanden für die ordnungsgemäße Durchführung einer Übung aufzubringende Kraft bereits innerhalb einer Beuge-Streck-Bewegung derart verändert, daß der Proband mit seiner Bewegung einer Vorgabekurve gerade noch folgen kann. Damit wird eine optimale Leistungsausbeute, ein optimaler Trainingseffekt erzielt.

Aus den von einer Datenverarbeitungsanlage, z.B. einem Rechner, aufgezeichneten Daten werden verschiedene Parameter zur Beurteilung der Übung ermittelt, beispielsweise die umgesetzte Hub- und Bremsleistung, die maximale Bewegungsamplitude, d.h. die durch die Bewegung des Probanden bewirkte maximale Winkel- bzw. Wegänderung.

Indem dem Probanden eine Kurvenform vorgegeben wird, die er durch geeignete Bewegung "nachzufahren" hat, und unter Aufsicht eines Therapeuten die vom Probanden geforderte Kraft ständig erhöht wird, läßt sich beispielsweise die Maximallast bestimmen, die der Proband kontrolliert handhaben kann. Diese kann dann beispielsweise bei der Festlegung der Trainingsparameter Anwendung finden.

Weitere Einzelheiten, Merkmale und Vorteile der vorliegenden Erfindung ergeben sich aus der folgenden Beschreibung mehrerer zeichnerisch dargestellter Ausführungsbeispiele. Es zeigen:
- Fig. 1: eine Anordnung zur Durchführung des erfindungsgemäßen Verfahrens;
- Fig. 2: das Hauptmenü eines zur Durchführung des erfindungsgemäßen Verfahrens geeigneten Rechnerprogramms mit zugehörigen Untermenüs;
- Fig. 3: ein Ablaufdiagramm für das erfindungsgemäße Diagnoseverfahren;
- Fig. 4: ein Ablaufdiagramm für die erfindungsgemäße Beweglichkeitsdiagnose;
- Fig. 5: ein Ablaufdiagramm für die erfindungsgemäße Maximalkraftdiagnose;
- Fig. 6: ein Ablaufdiagramm für die erfindungsgemäße Leistungsdiagnose;
- Fig. 7: ein Ablaufdiagramm für die erfindungsgemäße Koordinationsdiagnose;
- Fig. 8: ein Ablaufdiagramm für das erfindungsgemäße Trainingsverfahren;
- Fig. 9: das Ablaufdiagramm zur Generierung einer Vorgabekurve; und
- Fig. 10: eine Variante eines Trainingsgeräts zur Variation der vom Probanden aufzubringenden Kraft unter Verwendung einer schiefen Ebene.

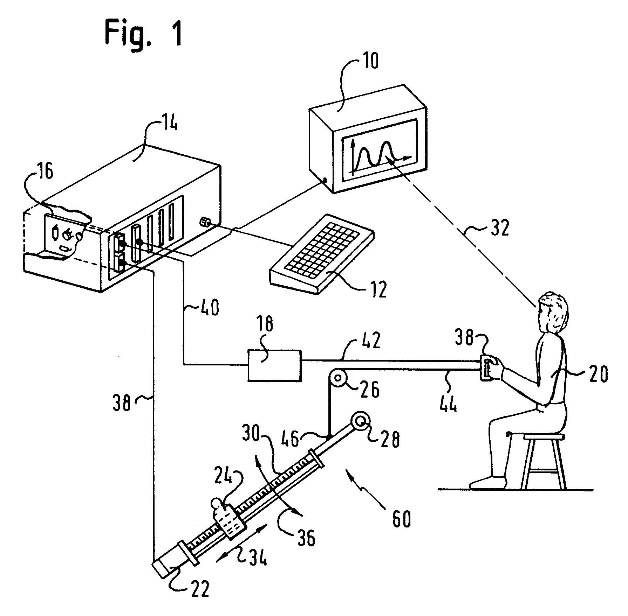

Figur 1 zeigt schematisch einen Aufbau zur Anwendung des erfindungsgemäßen Verfahrens. Dieser Aufbau umfaßt einen herkömmlichen Rechner 14, der um mindestens eine zusätzliche Steckkarte 16 erweitert worden ist, eine Tastatur 12, einen Bildschirm 10, sowie ein Trainingsgerät 60. In Figur 1 ist als Beispiel für ein Trainingsgerät ein Seilzuggerät abgebildet. Dieses Seilzuggerät 60 umfaßt einen um einen Drehpunkt 28 durch eine von einem Probanden 20 ausgeführte Beuge-Streck-Bewegung in Richtung des Doppelpfeils 36 zu bewegenden Hebel 30. Dazu zieht der Proband 20 beispielsweise an einem Handgriff 38, mit dem ein Seil 44 verbunden ist, das über eine Umlenkrolle 26 im Punkt 46 am Hebel 30 fixiert ist. In einer ersten Ausführungsform sei ein Motor 22 und eine Verbindung 38 zwischen diesem Motor 22 und dem Rechner 14 nicht vorhanden. Die vom Probanden für eine bestimmte Auslenkung des Hebels 30 aufzubringende Kraft kann durch Verschieben eines Gewichts 24 in Richtung des Doppelspfeils 34 auf dem Hebel 30 variiert werden. Neben dem Seil 44 ist ein Seil 42 mit dem Handgriff 38 verbunden. Das andere Ende des Seils 42 führt in einen Meßwertaufnehmer 18, beispielsweise einen Inkrementalgeber, der über eine Leitung 40 mit dem Rechner 14 verbunden ist. Die vom Probanden 20 ausgeführte Bewegung wird vom Meßwertaufnehmer 18 über das Seil 42 registriert, worauf der Meßwertaufnehmer 18 fortlaufend Daten erzeugt, die über die Leitung 40 in digitaler oder analoger Form an den Rechner 14 übertragen werden. Auf der Basis dieser Daten wird die vom Probanden 20 ausgeführte momentane Bewegungsamplitude bereits während der Bewegungsausführung, also synchron, am Bildschirm 10 in einem Auslenkungs-Zeit-Diagramm, wobei die Auslenkung beispielsweise der aufgrund einer Bewegung des Probanden bewirkten Längenänderung des Seils 42 entspricht, angezeigt. Wichtig ist, daß die Anzeige des Bildschirms 10 in der Blickrichtung 32 des Probanden 20 liegt, dieser also bereits während der Ausführung einer Bewegung eine dazu proportionale Größe mitgeteilt bekommt.

Dieses sogenannte Feedback kann auch auf andere Art und Weise hergestellt werden: Man kann beispielsweise dem Probanden 20 ein akustisches Signal zuführen, das aufgrund der von ihm ausgeführten Bewegung in der Tonhöhe oder in der Lautstärke variiert wird. In einer weiteren Ausführungsform kann dieses Feedback auf taktilkinestetischem Wege dem Probanden zugeführt werden. So kann beispielsweise dem Probanden 20 ein Berührungsdruck zugeführt werden, der sich proportional zu einer von ihm ausgeführten Bewegung ändert.

In einer weiteren Ausführungsform wird unter Verwendung mehrerer Meßwertaufnehmer nicht nur eine lineare Bewegung, sondern eine zwei- oder dreidimensionale Bewegung des Probanden 20 aufgezeichnet. Entsprechend werden dann zwei oder drei Kurven am Bildschirm angezeigt, die dann analysiert werden können. Dazu können, sofern die entsprechende Anzahl von Anschlüssen nicht auf einer zusätzlichen Steckkarte untergebracht werden kann, weitere zusätzliche Steckkarten im Rechner 14 untergebracht werden.

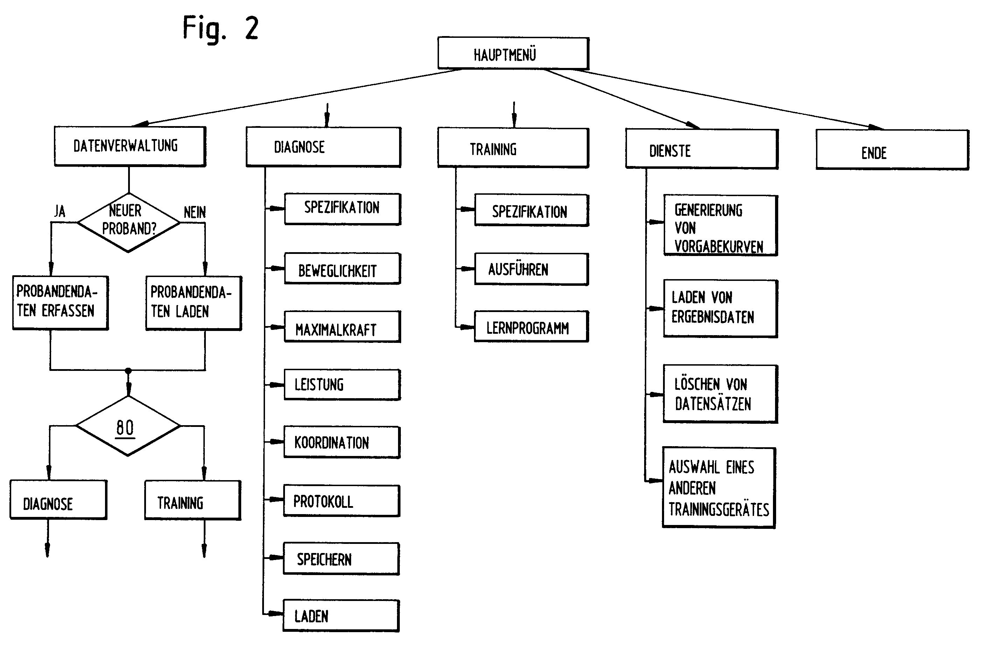

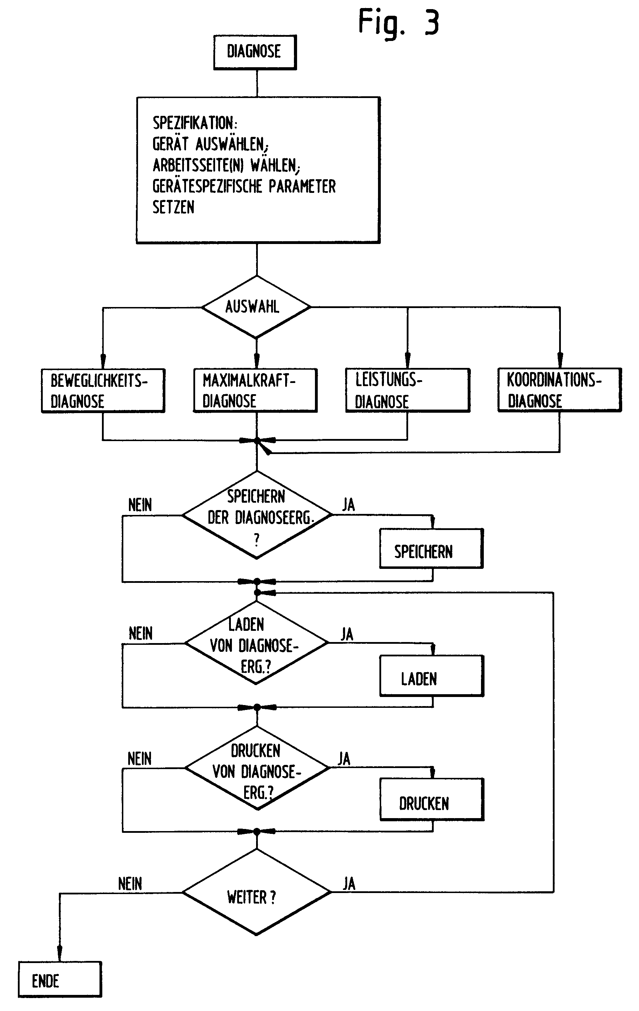

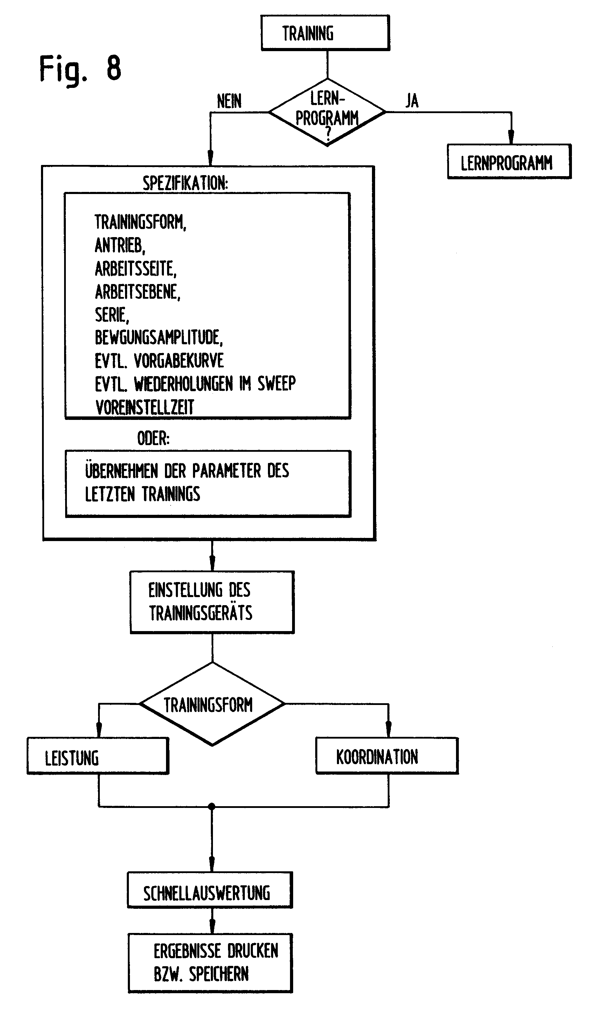

Das erfindungsgemäße Verfahren wird beispielsweise über ein Programm gesteuert, dessen Hauptmenü in Figur 2 schematisch dargestellt ist. In einem Untermenü für die Datenverwaltung wird zunächst abgefragt, ob es sich um einen neuen Probanden handelt. Wenn ja, werden die Probandendaten erfaßt, wenn nein, werden vorhandene Probandendaten geladen. Am Punkt 50 kann sich der Therapeut entscheiden, ob er ins Diagnose- oder Trainingsmenü verzweigen möchte. Im Untermenü Diagnose werden weitere Untermenüs angeboten: Spezifikation, Beweglichkeit, Maximalkraft, Leistung, Koordination, Protokoll, Speichern, Laden. Diese werden in der Beschreibung zu den Figuren 3 bis 7 genauer ausgeführt. Im Untermenü Training stehen die Menüs Spezifikation, Ausführen, Lernprogramm zur Verfügung. Diese werden mit Verweis auf Figur 8 genauer beschrieben. Im Untermenü Dienste stehen Menüs zur Verfügung zur Generierung von Vorgabekurven, zum Laden von Ergebnisdaten, zum Löschen von Datensätzen und zur Auswahl eines anderen Trainingsgerätes. Über ein weiteres Untermenü kann das Hauptmenü beendet werden.

In Figur 3 ist das Untermenü Diagnose genauer ausgeführt. Zunächst wird in einer Spezifikation ein Gerät ausgewählt, die Arbeitsseiten bestimmt und gerätespezifische Parameter gesetzt. Die Wahl der Arbeitsseite betrifft die Entscheidung, ob der Proband mit der linken, der rechten oder mit beiden Seiten arbeiten möchte. Im letzten Fall kann eine weitere Präzisierung vorgenommen werden, so kann angegeben werden, ob beide Seiten nacheinander, dabei erst links dann rechts oder beide Seiten nacheinander, dabei erst rechts dann links, oder beide Seiten gleichzeitig diagnostiziert werden sollen. Daran schließt sich die Auswahl des Diagnosetyps an. Zur Auswahl stehen Beweglichkeitsdiagnose, Maximalkraftdiagnose, Leistungsdiagnose und Koordinationsdiagnose. Nach der Durchführung einer dieser noch genauer zu beschreibenden Diagnosearten wird abgefragt, ob die Diagnoseergebnisse gespeichert werden sollen. Wenn "ja", werden sie gespeichert, wenn "nein" bzw. nach der Speicherung wird abgefragt, ob bereits gespeicherte Diagnoseergebnisse geladen werden sollen. Wenn "nein" bzw. nach dem Laden wird abgefragt, ob diese Diagnoseergebnisse gedruckt werden sollen. Wenn "nein" bzw. nach dem Drucken wird abgefragt, ob eine weitere Schleife durchlaufen werden soll. Wenn "nein", wird das Programm beendet, wenn "ja", wird zur Abfrage "Laden von Diagnoseergebnissen" zurückverzweigt.

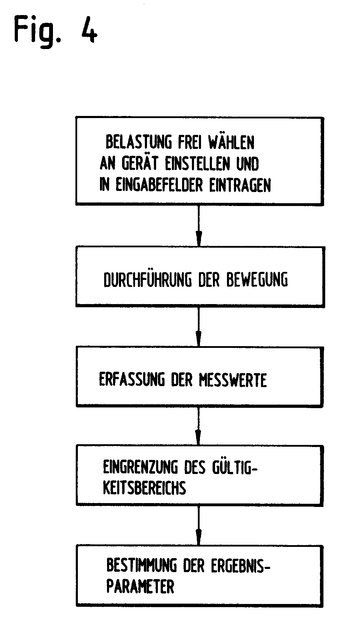

Figur 4 zeigt das Ablaufdiagramm der Beweglichkeitsdiagnose. Für die Beweglichkeitsdiagnose kann eine Maximalzahl von Versuchen mit je einer Streck-Beuge-Bewegung, von sogenannten Wiederholungen, ausgeführt werden. Festgestellt werden die Bewegungsamplitude sowie die Zeiten für die Streck-, die Beuge- und die Streck-Beuge-Bewegung. Hier wird zunächst eine Belastung frei gewählt, am Trainingsgerät eingestellt und in den entsprechenden Eingabefeldern am Rechner unter Verwendung der Tastatur 12 eingetragen. In einer bevorzugten Ausführungsform wird daraufhin am Rechnerbildschirm ein entsprechendes Diagramm abgebildet, in dem die Abszisse der Zeit und die Ordinate der Auslenkung, beispielsweise des Seils 42 in Fig. 1 entspricht. Während der sich anschließenden Durchführung der Bewegung am Trainingsgerät werden entsprechende Meßwerte erfaßt und, wie bereits erwähnt, als Kurvenzug am Bildschirm angezeigt. Nach Beendigung der Meßwerterfassung kann der Gültigkeitsbereich, d. h. der zur Auswertung zu verwendende Teil der angezeigten Kurve vom Therapeuten eingegrenzt werden. Auf Grundlage der damit erhaltenen Meßkurve werden daraufhin verschiedene Ergebnisparameter vom Rechner bestimmt. Beispielsweise werden, wie bereits erwähnt, die Bewegungsamplitude, die Zeit für die Flexion bzw. die Extension, die Zeit vom Beginn der Bewegung bis zum Erreichen der maximalen Auslenkung bzw. die Zeit von der maximalen Auslenkung bis zum Erreichen des Ursprungszustands ermittelt.

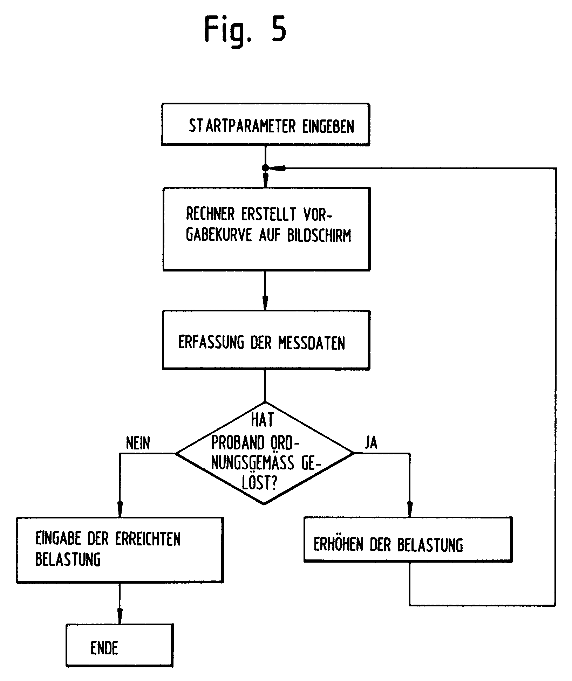

Bei der Maximalkraftdiagnose muß der Proband bei sich steigernden Lasten eine vorgegebene Kurve mit seiner Bewegung mehrmals nachvollziehen. Der Therapeut erhöht die Last so lange, bis der Proband die vorgegebene Kurve nicht mehr nachvollziehen kann. Figur 5 zeigt das zugehörige Ablaufdiagramm. Zunächst werden über die Tastatur 12 Startparameter eingegeben, wie z.B. die vom Probanden zu erreichende Auslenkungsamplitude oder die Zeit für eine Wiederholung. Daraufhin wird vom Rechner eine Vorgabekurve berechnet und auf dem Bildschirm dargestellt. Danach beginnt der Proband mit der Ausführung seiner Übung und versucht dabei, seine Bewegung derart zu koordinieren, daß die dadurch bewirkte simultane Anzeige, die der aufgrund seiner Bewegung bewirkten Auslenkung proportional ist, am Bildschirm möglichst exakt mit der Vorgabekurve zusammenfällt. Die dabei entstehenden Meßdaten werden aufgezeichnet. Nach Durchführung der Übung hat der Therapeut die Frage zu beantworten, ob der Proband seine Aufgabe ordnungsgemäß gelöst hat, d.h. ob seine Kraftfähigkeit für die koordinierte isokinetische Bewegungsausführung bei der eingestellten Last ausreichend war. Ist dies der Fall, so wird die Belastung am Trainingsgerät automatisch oder vom Therapeuten erhöht. Nach einer kurzen Pause wird auf dem Bildschirm das Koordinatensystem mit der Vorgabekurve neu gezeichnet und alles läuft wie beim ersten Mal ab. Ist eine Last erreicht, bei welcher der Proband die Bewegungsaufgabe nicht mehr ordnungsgemäß erfüllen kann, wird der Therapeut zur Eingabe der erreichten Belastung aufgefordert, bei welcher der Proband die Bewegungsaufgabe noch erfüllt hat.

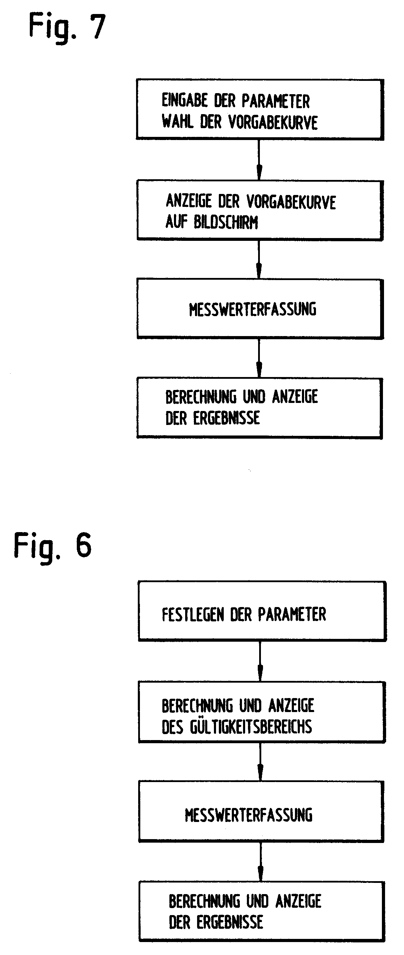

In Figur 6 ist schematisch der Ablauf der Leistungsdiagnose dargestellt. Zur Leistungsdiagnose muß der Proband mit einer gegebenen Bewegungsamplitude und Last über eine festgesetzte Zeit Beuge-Streck-Bewegungen durchführen. Zunächst müssen die entsprechenden Parameter festgelegt werden. Diese werden am Rechner eingegeben und am Trainingsgerät eingestellt. In einer vorteilhaften Ausführungsform werden in das Koordinatensystem, dessen Abszisse die Zeit und dessen Ordinate die Auslenkung bezeichnet, neben den Hilfslinien zur Begrenzung der Bewegungsamplitude zwei weitere Hilfslinien bei 10 % und 90 % der voreingestellten Amplitude berechnet und angezeigt. Anschließend werden die vom Probanden 20 bewirkten Meßwerte erfaßt und simultan angezeigt. Schließlich werden die Ergebnisse, wie z.B. die Anzahl der Gesamtwiederholungen, die Anzahl von gültigen Wiederholungen, die Arbeit, die Hubleistung und die Bremsleistung berechnet und angezeigt.

Bei der in Figur 7 gezeigten Koordinationsdiagnose soll der Proband eine vom Therapeuten ausgewählte, beispielsweise sinusförmige Vorgabekurve durch seine Bewegungsausführung nachzeichnen. Es wird eine bestimmte Anzahl von Wiederholungen gefordert. Dazu werden zunächst Startparameter, wie Amplitude, Zeit pro Wiederholung und Last eingegeben und eine Vorgabekurve gewählt. Daraufhin wird die gewählte Vorgabekurve in einem Koordinatensystem am Bildschirm angezeigt, worauf der Proband mit seiner Bewegung beginnt und die dabei entstehenden Meßwerte erfaßt werden. Anschließend werden als Ergebnis beispielsweise die über mehrere Wiederholungen gemittelten Ist-Kurven im Vergleich zur Soll-Kurve und die Kurven der statistischen Streuung angezeigt.

Die erhaltenen Diagnoseergebnisse können, wie in Figur 3 gezeigt, gespeichert, geladen bzw. gedruckt werden.

In Figur 8 ist das Ablaufdiagramm des Trainingsmenüs dargestellt. Die in der Spezifikation geforderten Eingaben beschreiben das Trainingsprogramm hinsichtlich seiner Applikation auf bestimmte skelettmotorische Einheiten, seines energetischen Umfangs und seiner Art des Feedbacks. Zunächst kann der Anwender entscheiden, ob er ein Lernprogramm ausführen will oder nicht. Im Falle "ja" wird zum Lernprogramm verzweigt. Die Arbeit mit dem Lernprogramm soll dem Probanden ein Gefühl für das Bewegungs-Feedback vermitteln. Er soll ohne Einschränkungen und Vorgaben Beuge-Streck-Bewegungen ausführen und dabei den Verlauf der Feedback-Kurve beobachten. Im Falle "nein" ist zunächst das Trainingsprogramm zu spezifizieren. Dazu sind mehrere Parameter zu bestimmen, z.B. die noch genauer zu beschreibende Trainingsform, die Art des Antriebs, der die Gelenkantriebe bezeichnet, die mit dem Training angesprochen werden sollen, die Arbeitsseite, die Arbeitsebene, die bei ebenen Bewegungen die Ebene kennzeichnet, in der die Bewegung ausgeführt wird (sagittal, frontal und transversal), die Serie, die die zyklische Wiederholung der vorgeschriebenen Bewegung entsprechend der gegebenen Anzahl von Wiederholungen ohne Pause zwischen den einzelnen Wiederholungen kennzeichnet, die Bewegungsamplitude, die bezogen auf den Bewegungsbeginn immer bei Null beginnt, und den maximalen Wert des Bewegungsbereichs kennzeichnet, eventuell eine Vorgabekurve, die bei bestimmten Trainingsformen anzugeben ist, eventuell die Anzahl der Wiederholungen in einem Sweep, d.h. wieviele Wiederholungen einer Serie auf einmal am Bildschirm angezeigt werden sollen, bevor die Wiederholungen des nächsten Sweeps am Bildschirm angezeigt werden, wobei die Anzahl Wiederholungen in einer Serie gleich dem Produkt aus Anzahl der Wiederholungen pro Sweep und Anzahl der Sweeps ist, und schließlich gegebenenfalls eine Voreinstellzeit, d.h. ein Zeitintervall zu Beginn eines jeden Sweeps, in dem keine Bewegung ausgeführt wird. Während dieser Zeit soll sich der Proband auf die Bewegungsausführung vorbereiten. Alternativ können die während des letzten Trainings spezifizierten Parameter übernommen werden. Anschließend werden die das Trainingsgerät betreffenden, während der Spezifikation angegebenen Parameter am Trainingsgerät eingestellt. Je nach spezifizierter Trainingsform wird anschließend in das Leistungstraining oder das Koordinationstraining verzweigt.

Bei der Trainingsform Leistung entspricht die Zeitachse der festgelegten Trainingszeit für eine Serie. Auf der Ordinate sind bei Null und der festgesetzten Bewegungsamplitude horizontale Hilfslinien sowie bei 10 % und 90 % der Bewegungsamplitude weitere horizontale Hilfslinien eingezeichnet. Die äußeren Linien begrenzen den Bewegungsbereich, während die inneren Linien den Gültigkeitsbereich begrenzen. Der Proband soll seine Beuge-Streck-Bewegungen so ausführen, daß die aufgrund seiner Bewegung am Bildschirm angezeigte Kurve die äußeren Linien gerade erreicht und nicht überschreitet, auf jeden Fall aber die inneren Linien über- bzw. unterschreitet.

Bei der Trainingsform Koordination wird zusätzlich zu den eben genannten vier Linien noch eine vom Therapeuten ausgewählte Vorgabekurve in das Koordinatensystem eingezeichnet, die der Proband mit seiner Bewegung möglichst genau "nachfahren" soll. In einer weiteren Ausführungsform ist eine automatische Starterkennung implementiert, d.h. die Meßdatenerfassung und Feedback-Darstellung beginnt erst mit dem "Anrucken", d. h. mit einer ersten Bewegung des Probanden. Ansonsten wird die Meßdatenerfassung und Feedback-Darstellung nach Ablauf eines Countdowns gestartet.

Neben den hier beispielhaft vorgestellten Trainingsformen sind selbstverständlich weitere Trainingsformen denkbar.

Es schließt sich eine Schnellauswertung an, bei der als Ergebnis beispielsweise die Bewegungsamplitude, die mittlere Hubleistung bzw. die mittlere Bremsleistung zu jeder Wiederholung der Serie und die verrichtete Arbeit berechnet und am Bildschirm dargestellt werden. Darüber hinaus läßt sich die Zeit zu jeder Wiederholung der Serie bei der Trainingsform Leistung bzw. ein Vergleich der Soll-Kurve zur mittleren Ist-Kurve und die Streuung der Ist-Kurven bei der Trainingsform Koordination bestimmen und am Bildschirm anzeigen. Diese Ergebnisse können gedruckt bzw. gespeichert werden.

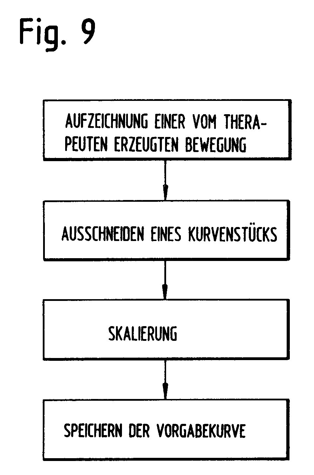

In Figur 9 ist die Vorgehensweise zur Generierung von Vorgabekurven aufgeführt. Zunächst wird eine vom Therapeuten selbst ausgeführte Bewegung erfaßt und im Koordinatensystem der Feedback-Kurve am Bildschirm angezeigt. Nach dem Aufzeichnen der Kurve wird der gültige Bereich eingegrenzt. Anschließend kann dieser gültige Bereich durch die Eingabe von "Zeit für eine Wiederholung" und "Bewegungsamplitude" auf die gewünschte Größe skaliert werden. Die resultierende Vorgabekurve kann anschließend abgespeichert werden.

Eine weitere Ausführungsform vorliegender Erfindung kann Figur 1 entnommen werden. Hierbei ist nun ein mit dem Hebel 30 verbundener Motor 22, beispielsweise ein Elektromotor, über die Leitung 38 mit einer weiteren Zusatzplatine oder, soweit möglich, mit der Zusatzplatine 16 des Rechners verbunden. Mit einer derart erweiterten Anordnung läßt sich eine Adaption an die aktuelle Leistungsfähigkeit des Probanden 20 herbeiführen.

Wird über die Kette aus Handgriff 38, Seil 42, Meßwertaufnehmer 18, Leitung 40 und Rechner 14 festgestellt, daß der Proband 20 die für eine ordnungsgemäße Durchführung einer Übung erforderliche Kraft nicht aufbringen kann, so kann auf zweierlei Arten an die tatsächliche Kraft des Probanden adaptiert werden: Zum einen wird durch Verschieben des Gewichts 24 auf dem Hebel 30 durch den vom Rechner 14 gesteuerten Motor 22 in Richtung des Doppelpfeils 34 eine Änderung des vom Gewicht 24 bewirkten Drehmoments verursacht und somit eine Änderung der vom Probanden 20 zur Überwindung dieses Drehmoments aufzubringenden Kraft. Andererseits können bei konstantem Drehmoment, d.h. das Gewicht 24 bleibt bezüglich des Hebels 30 auf einer festen Position, die Parameter der Vorgabekurve, beispielsweise die zur Ausführung einer Wiederholung erlaubte Zeit, oder die zu erreichende Auslenkung, während der Durchführung einer Übung bereits innerhalb einer Beuge-Streck-Bewegung an die vom Probanden 20 individuell aufzubringende Kraft angepaßt werden. Hiermit kann präzise an der Leistungsgrenze eines Probanden gearbeitet werden.

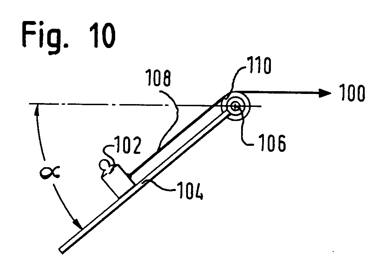

Eine weitere Möglichkeit, die vom Probanden 20 aufzubringende Last bereits während der Durchführung einer Wiederholung an die tatsächliche Leistungsfähigkeit des Probanden anzupassen, ist in Figur 10 dargestellt. Ein Gewicht 102 befindet sich auf einer schiefen Ebene 104, die gegenüber der Horizontalen einen Winkel α einnimmt. Ein Proband (nicht gezeigt) zieht an einem Seil 108, das über eine Umlenkrolle 106 läuft, in Richtung 100. Zur Anpassung an die tatsächliche Leistungsfähigkeit des Probanden wird der Winkel α während der Durchführung einer Übung derart verändert, daß der Proband die Übung ordnungsgemäß absolvieren kann.

In weiteren Ausführungsformen kann die vom Probanden aufzubringende Kraft, nicht nur wie hier ausgeführt gegen die Erdanziehungskraft, sondern auch gegen eine Federkraft oder eine motorisch bewirkte Kraft gerichtet sein.

## Patentansprüche

1. Verfahren zur Diagnose der Fähigkeiten des menschlichen Bewegungsapparates und des kontrollierten und dosierten Trainings dieser Fähigkeiten unter Verwendung eines um mindestens eine Steckkarte (16) erweiterten herkömmlichen Rechners (14), eines herkömmlichen Trainigsgeräts (60) und eines Meßwertaufnehmers (18), folgende Schritte umfassend:
- Einstellen von Startparametern am Trainingsgerät (16);
- Eingeben dieser Werte am Rechner (14);
- Verbinden des mindestens einen Meßwertaufnehmers (18) mit dem Trainingsgerät (60) und mit dem Rechner (14);
- Fortlaufende Übertragung (40) der sich aus der von einem Probanden (20) während seiner am Trainingsgerät (60) ausgeführten Bewegung ergebenden, vom mindestens einen Meßwertaufnehmer (18) in Abhängigkeit dieser Bewegung des Probanden (20) erzeugten Daten an die zusätzliche Steckkarte (16) des Rechners (14);
- Vermitteln (10,32) einer aus diesen, der momentanen Änderung gegenüber einem Grundzustand entsprechenden Daten abgeleiteten Größe simultan zu seiner Bewegung an den Probanden (20);
- Auswertung dieser Daten im Rechner (14) durch Berechnung verschiedener zur Diagnose und Kontrolle bzw. Dosierung des Trainings geeigneten Parameter;
- Anzeige der Ergebnisse dieser Auswertung am Rechnerbildschirm (10).

2. Verfahren nach Anspruch 1 dadurch gekennzeichnet, daß einer oder mehrere herkömmliche Rechner, ein oder mehrere herkömmliche Trainingsgeräte und eine oder mehrere zusätzliche Steckkarten verwendet werden.

3. Verfahren nach Anspruch 1 oder 2 dadurch gekennzeichnet, daß die der momentanen Änderung gegenüber einem Grundzustand entsprechenden Daten dem Probanden durch Anzeige auf einem vom Probanden während der Durchführung einer Übung einsehbaren Bildschirm vermittelt werden.

4. Verfahren nach Anspruch 1 oder 2 dadurch gekennzeichnet, daß die momentane Änderung gegenüber einem Grundzustand dem Probanden durch ein entsprechend dieser momentanen Änderung in der Lautstärke variiertes Signal vermittelt wird.

5. Verfahren nach Anspruch 1 oder 2 dadurch gekennzeichnet, daß die momentane Änderung gegenüber einem Grundzustand dem Probanden durch ein entsprechend dieser momentanen Änderung in der Tonhöhe variiertes Signal vermittelt wird.

6. Verfahren nach Anspruch 1 oder 2 dadurch gekennzeichnet, daß die momentane Änderung gegenüber einem Grundzustand Auslenkung dem Probanden durch ein entsprechend dieser momentanen Änderung variiertes Drucksignal vermittelt wird.

7. Verfahren nach einem der Ansprüche 3 bis 6 dadurch gekennzeichnet, daß die vom Probanden im Rahmen der von ihm ausgeführten Bewegung aufgebrachte Kraft gegen die Schwerkraft gerichtet ist.

8. Verfahren nach einem der Ansprüche 3 bis 6 dadurch gekennzeichnet, daß die vom Probanden im Rahmen der von ihm ausgeführten Bewegung aufgebrachte Kraft gegen eine Federkraft gerichtet ist.

9. Verfahren nach einem der Ansprüche 3 bis 6 dadurch gekennzeichnet, daß die vom Probanden im Rahmen der von ihm ausgeführten Bewegung aufgebrachte Kraft gegen eine motorisch erzeugte Kraft gerichtet ist.

10. Verfahren nach einem der vorhergehenden Ansprüche dadurch gekennzeichnet, daß das Trainingsgerät ein Kniestrecker ist.

11. Verfahren nach einem der Ansprüche 1 bis 9 dadurch gekennzeichnet, daß das Trainingsgerät ein Seilzuggerät ist.

12. Verfahren nach einem der vorhergehenden Ansprüche dadurch gekennzeichnet, daß der Meßwertaufnehmer ein Inkrementalgeber ist.

13. Verfahren nach einem der vorhergehenden Ansprüche dadurch gekennzeichnet, daß die Variation der vom Probanden aufzubringenden Kraft durch Variation der Masse eines vom Probanden zu bewegenden Teils des Trainingsgeräts erreicht wird.

14. Verfahren nach einem der vorhergehenden Ansprüche dadurch gekennzeichnet, daß zur die zur Bewegung einer konstant bleibenden Masse nötige Kraft veränderbar ist.

15. Verfahren nach Anspruch 14 dadurch gekennzeichnet, daß die zur Bewegung nötige Kraft über eine Änderung des Anstellwinkels einer schiefen Ebene verändert wird.

16. Verfahren nach Anspruch 14 dadurch gekennzeichnet, daß die zur Bewegung nötige Kraft über eine Hebelarmänderung verändert wird.

17. Verfahren nach einem der vorhergehenden Ansprüche dadurch gekennzeichnet, daß einer der Startparameter die maximale Bewegungsamplitude ist.

18. Verfahren nach einem der vorhergehenden Ansprüche dadurch gekennzeichnet, daß einer der Startparameter die Masse des vom Probanden zu bewegenden Teils des Trainingsgeräts ist.

19. Verfahren nach Anspruch 15 dadurch gekennzeichnet, daß einer der Startparameter der Anstellwinkel der schiefen Ebene ist.

20. Verfahren nach Anspruch 16 dadurch gekennzeichnet, daß einer der Startparameter der Hebelarm ist, an dem ein Proband eine Masse zu bewegen hat.

21. Verfahren nach einem der vorhergehenden Ansprüche dadurch gekennzeichnet, daß, bevor vom Probanden eine Bewegung ausgeführt wird, ein auswählbare Vorgabekurve am Bildschirm angezeigt wird.

22. Verfahren nach Anspruch 21 dadurch gekennzeichnet, daß der aktuelle, vom Probanden aufgrund seiner Bewegung entstehende Kurvenzug zusätzlich zur Vorgabekurve am Bildschirm angezeigt wird.

23. Verfahren nach Anspruch 21 oder 22 dadurch gekennzeichnet, daß eine gemittelte Ist-Kurve aus den vom Probanden geleisteten Bewegungen bestimmt wird.

24. Verfahren nach Anspruch 23 dadurch gekennzeichnet, daß aus den gemittelten Ist-Kurven und der Vorgabekurve eine statistische Streuung berechnet wird.

25. Verfahren nach einem der Ansprüche 21 bis 24 dadurch gekennzeichnet, daß Mittel zur Erstellung und Speicherung von Vorgabekurven vorhanden sind.

26. Verfahren nach einem der Ansprüche 21 bis 24 dadurch gekennzeichnet, daß der Proband seine Bewegung derart auszuführen hat, daß die dadurch bewirkte Anzeige, die der aufgrund seiner Bewegung bewirkten Änderung gegenüber einem Grundzustand proportional ist, am Bildschirm möglichst exakt mit der Vorgabekurve zusammenfällt.

27. Verfahren nach Anspruch 26 dadurch gekennzeichnet, daß die Vorgabekurve eine Sinusfunktion darstellt.

28. Verfahren nach einem der vorhergehenden Ansprüche dadurch gekennzeichnet, daß der durch die Bewegung einer einzelnen Extremität bewirkte Kurvenzug am Bildschirm angezeigt wird.

29. Verfahren nach einem der Ansprüche 1 bis 27 dadurch gekennzeichnet, daß gleichzeitig die von zwei nacheinander bewegten Extremitäten bewirkten Kurvenzüge am Bildschirm angezeigt werden.

30. Verfahren nach einem der Ansprüche 1 bis 27 dadurch gekennzeichnet, daß gleichzeitig die von zwei Extremitäten bewirkten Kurvenzüge am Bildschirm angezeigt werden.

31. Verfahren nach einem der vorhergehenden Ansprüche dadurch gekennzeichnet, daß bei der Auswertung der Daten im Rechner die maximale aus der Bewegung des Probanden resultierende Änderung gegenüber einem Grundzustand bestimmt wird.

32. Verfahren nach Anspruch 31 dadurch gekennzeichnet, daß die Änderung eine Winkeländerung darstellt.

33. Verfahren nach Anspruch 31 dadurch gekennzeichnet, daß die Änderung eine Wegänderung darstellt.

34. Verfahren nach einem der vorhergehenden Ansprüche dadurch gekennzeichnet, daß die Meßdatenerfassung nach Ablauf eines Countdowns beginnt.

35. Verfahren nach einem oder mehreren der Anspüche 1 bis 33 dadurch gekennzeichnet, daß die Meßdatenerfassung mit einer ersten Bewegung (Anrucken) des Probanden beginnt.

36. Verfahren nach einem oder mehreren der vorhergehenden Ansprüche dadurch gekennzeichnet, daß für die vom Probanden bewirkte Amplitudenänderung ein erlaubter unterer Toleranzbereich der Bereich zwischen 0% und 10% und als erlaubter oberer Toleranzbereich der Bereich zwischen 90% und 100% eines Maximalwerts am Bildschirm angezeigt wird.

37. Verfahren nach Anspruch 36 dadurch gekennzeichnet, daß der Maximalwert aus den Ergebnissen vorhergehender Übungen bestimmt wird.

38. Verfahren nach einem der vorhergehenden Ansprüche dadurch gekennzeichnet, daß die vom Probanden maximal ordnungsgemäß zu dosierende Kraft bestimmt wird.

39. Verfahren nach Anspruch 38 dadurch gekennzeichnet, daß die vom Probanden ordnungsgemäß zu bewegende maximale Last bestimmt wird.

40. Verfahren nach einem der vorhergehenden Ansprüche dadurch gekennzeichnet, daß die vom Probanden geleistete Arbeit, die Hubleistung und die Bremsleistung ermittelt wird.

41. Verfahren nach Anspruch 40 dadurch gekennzeichnet, daß vom Probanden bei vorgegebener Last eine bestimmte Anzahl von Wiederholungen, d.h. Beuge-Streck-Bewegungen, mit einer Amplitude innerhalb vorgebbarer Grenzen und bestimmter Zeitdauer geleistet werden muß.

42. Verfahren nach Anspruch 40 dadurch gekennzeichnet, daß vom Probanden bei vorgegebener Last und vorgebener Zeit möglichst viele Wiederholungen mit einer Amplitude innerhalb vorgebbarer Grenzen ausgeführt werden müssen.

43. Verfahren nach Anspruch 36 dadurch gekennzeichnet, daß die Anzahl der gültig ausgeführten Wiederholungen am Bildschirm angezeigt wird.

44. Verfahren nach einem der vorhergehenden Ansprüche dadurch gekennzeichnet, daß die Zeit für Flexion und die Zeit für Extension bestimmt werden.

45. Verfahren nach einem der vorhergehenden Anspüche dadurch gekennzeichnet, daß die Zeit von einem Grundzustand bis zum Erreichen des Maximums der Bewegungsamplitude und die Zeit vom Maximum der Bewegungsamplitude bis zum Eintritt des Grundzustands ermittelt wird.

46. Verfahren nach einem der vorhergehenden Ansprüche dadurch gekennzeichnet, daß der auszuwertende Anteil des vom Probanden bewirkten Kurvenzugs angegeben werden kann.

47. Verfahren nach einem der vorhergehenden Anspüche dadurch gekennzeichnet, daß die Ergebnisse der Auswertung gespeichert, ausgegeben und wieder geladen werden können.

48. Verfahren nach einem der vorhergehenden Ansprüche dadurch gekennzeichnet, daß Probandendaten verwaltet werden können.

49. Verfahren nach einem der vorhergehenden Ansprüche dadurch gekennzeichnet, daß die vom Probanden für eine bestimmte Bewegungsamplitude aufzubringende Kraft bereits innerhalb einer Wiederholung soweit verändert werden kann, daß der Proband die geforderte Bewegungsamplitude erreicht.

50. Verfahren nach Anspruch 49 dadurch gekennzeichnet, daß diese Änderung vom Rechner unter Verwendung der vom Meßwertaufnehmer gelieferten Daten bewirkt wird.

51. Verfahren nach Anspruch 49 oder 50 dadurch gekennzeichnet, daß der Rechner den Hebelarm, an dem der Proband eine Last zu bewegen hat, ändert.

52. Verfahren nach Anspruch 49 oder 50 dadurch gekennzeichnet, daß der Rechner den Anstellwinkel einer schiefen Ebene, auf der der Proband eine Last zu bewegen hat, ändert.

53. Verfahren nach Anspruch 49 oder 50 dadurch gekennzeichnet, daß der Rechner die motorisch erzeugte Kraft, der der Proband entgegenzuwirken hat, ändert.

54. Verfahren nach Anspruch 9 dadurch gekennzeichnet, daß die vom Probanden erzeugte, gegen die motorisch erzeugte Kraft gerichtete Gegenkraft registriert und im Rechner ausgewertet wird.
